(19) **Europäisches Patentamt / European Patent Office / Office européen des brevets**

(11) **EP 2 292 284 B1**

(12) **EUROPEAN PATENT SPECIFICATION**

(45) Date of publication and mention
of the grant of the patent:
**26.02.2014 Bulletin 2014/09**

(51) Int Cl.:
**A61M 1/16** *(2006.01)*    *A61M 1/34* *(2006.01)*

(21) Application number: **09011353.1**

(22) Date of filing: **04.09.2009**

(54) **Kidney substitution treatment machine**

Nierenersatzbehandlungsmaschine

Machine de traitement de remplacement de rein

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**09.03.2011 Bulletin 2011/10**

(73) Proprietor: **B. Braun Avitum AG**
**34212 Melsungen (DE)**

(72) Inventors:
• **Castellarnau, Alex**
**34212 Melsungen (DE)**

• **Ahrens, Jörn**
**34225 Baunatal (DE)**
• **Moll, Stefan**
**34212 Melsungen (DE)**

(74) Representative: **Arth, Hans-Lothar**
**ABK Patent Attorneys**
**Jasminweg 9**
**14052 Berlin (DE)**

(56) References cited:
**EP-A1- 1 348 457**    **EP-A1- 2 005 982**
**WO-A1-99/62574**    **US-A1- 2001 004 523**
**US-A1- 2005 045 540**    **US-B1- 6 702 774**

## Description

The invention relates to a device to store and display a history of Kt/V or other adequacy parameters trends in a machine able to perform kidney substitution treatments. The stored trends can be evaluated against the ongoing treatment offering some parameters, which may be used by the medical staff to assess said ongoing treatment.

Dialysis adequacy is the topic that has got and gets more attention when one thinks about patient outcome. In order to estimate dialysis adequacy one needs a parameter establishing a relation between dialysis dosage and patient outcome. The most accepted parameter to estimate the quantity of dialysis delivered or dosage is the Kt/V, where K is the effective clearance for urea, t is the treatment time and V is the urea distribution volume which matches the total body water.

The NCDS (National Cooperative Dialysis Study) and the HEMO study found, after analyzing a large patient group, that morbidity and mortality in end stage renal disease (ESRD) was strongly correlated with the Kt/V value or dialysis dose. Data obtained from these studies resulted in guidelines regarding hemodialysis treatments, which demand a minimum dose of Kt/V-1.2 for non-diabetic patients and 1.4 for diabetics (DOQ) guidclines). It is worthy to point out that a morbidity decrease not only improves the patient well-being, but also reduces Significantly the medical costs as the patient requires less care.

The need of a reliable and cost effective method to monitor the Kt/V and by extension control dialysis adequacy and morbidity, is therefore easily understood.

In the Kt/V calculation, the main problems are K and V estimation along with the multi-compartment urea kinetics. V can be estimated by bioimpedance, anthropometric measurements or applying the urea kinetic model (UKM), all these methods have a certain degree of error. K can be estimated so far by measuring the urea blood concentration before and after the treatment or by monitoring inlet and outlet conductivity changes in the dialysate side.

13lood samples method is the reference one. After taking the blood samples and applying either UKM or Daugirdas formula a single pool Kt/V (spKt/V) is estimated, further,

Daugirdas second generation formulas should be used to get an equilibrated Kt/V (eKt/V) which accounts for the urea rebound caused by the fact that urea kinetic's does not follow a single pool model but a multi-compartment one. This method has two main problems: it is not possible to know whether the treatment is adequate or not before it finishes, therefore it is not possible to perform any action to improve the situation; it is not an easy to apply method: sampling time is very important to get an accurate value, and the medical stuff must send the samples to the lab, wait for the results and calculate Kt/V values with the help of a computer. These facts result on a monthly basis Kt/V measurements in best case, which means that in worst case scenario a patient might be under-dialyzed for one whole month.

Conductivity methods are based on the observation that sodium clearance is almost equal to urea clearance and that the relationship between dialysate conductivity and dialysate sodium concentration can be considered linear on the temperature range of interest. Therefore it is possible to get urea clearance by measuring the sodium diffusion transport through the membrane in the dialyzer.

It is important to introduce the concept of Dialysance, as it slightly differs from Clearance:

Clearance is defined as the ratio between transport rate and concentration multiplied by flow, and it is applicable when the diffusing substance is on the blood side but not on the dialysate, that is the case for urea.

Dialysance is defined as the ratio between transport rate and concentration gradient multiplied by flow, and it is applicable when the diffusing substance is in both dialyzer sides. When one applies conductivity methods to measure urea Clearance, one actually measures sodium Dialysance (Depner T, Garred I.. Solute transport mechanisms in dialysis. Hörl W, Koch K, Lindsay R, Ronco C, Winchester JF, editors. Replacement of renal function by dialysis, 5th ed. Kluwer academic publishers, 2004:73-91).

During conductivity based clearance measurements, a dialysate inlet conductivity different to the blood one is produced, which results in a net transfer of sodium either from blood to dialysate or from dialysate to blood due to the generated gradient. There are currently several methods which are applied in the industry:

In a first method a one-step conductivity profile is performed; in a second method a two-step conductivity profile is performed; and in a third method an integration of conductivity peaks is used. (Polaschegg HD, Levin NW. Hemodialysis machines and monitoris. Hörl W, Koch K, Lindsay R, Ronco C, Winchester JF, editors. Replacement of renal function by dialysis, 5th ed. Kluwer academic publishers, 2004:414-418). The main advantage of this approach is that it is relatively easy to implement and cost effective as it only needs an extra conductivity/temperature sensor downstream the dialyzer. It offers Kt/V measurements during the treatment allowing the medical staff to react and perform some actions in case the treatment is not going as it should. However, conductivity based methods have also some limitations: they can induce some sodium load in the patient during the measurement; they are not useful to obtain other interesting parameters like nPCR. or TRU. The maximum measurement frequency offered so far by the industry is about 20 minutes, which means that in worst case scenario the patient could be under-dialyzed for 20 minutes. And although there are some publications claiming it, so far, conductivity methods haven't been applied with enough reliability to hemofiltration or hemodiafiltration treatments.

Another method to estimate hemodialysis adequacy is by direct measurement of the waste products (urea) concentration in the effluent dialysate, this method assumes that the evolution of urea concentration over the time in the dialysate side is proportional to the one in the blood, therefore the slope of the line obtained after applying the natural logarithm to the registered concentration values over the time will be the same on both sides: dialysate side and blood side. And by definition such slope is K/V, which multiplied by the therapy time results in the Kt/V value.

There arc two different methods available to measure online the concentration of waste products in effluent dialysate: Urea sensors and UV spectrophotometry.

The limitations of the urea sensors are well known. Recent works carried out by Fridolin I. et al (I. Fridolin, M. Magnusson, L-G. Lindberg. On-line monitoring of solutes in dialysate using absorption of ultraviolet radiation: Technique description. The International Journal of Artificial Organs. Vol. 25, no. 8, 2002, pp. 748-761) and Uhlin F. (Uhlin F. Haemodialysis treatment monitored online by ultra violet absorbance. Linköping University Medical Dissertations n° 962. Department of Medicine and Care Division of Nursing Science & Department of Biomedical Engineering. 2006.) have shown that UV spectrophotometry is a reliable and cost affordable method to monitor waste products in effluent dialysate, Additionally, the European Patent EP1083948B1 describes a sensor coupled with the dialysate flow system of a dialysis machine, which is actually an UV spectrophotomcter measuring UV absorbance of UV absorbing products in spent dialysate.

By direct measuring waste products in spent dialysate, a curve representing the concentration or absorbance decay of uremic compounds over the whole dialysis period is obtained. These curves sightly reflect the course of the treatment and manifest patient based haemodynamic singularities, which influence the dialysis efficiency. The obtained concentration-curves could be saved either in the dialysis machine or in a patient card. During a dialysis procedure, the saved curves could be displayed by user request. Visual comparisons between the ongoing and the previously recorded therapies could help the physician to identify problems Moreover, a comparison algorithm could generate some parameters to quantify these discrepancies. A signal could be triggered when the differences get over a certain threshold to draw the attention of the medical staff, and help them on deciding if an action is necessary.

The problem of this invention is to provide a reliable device to record and display concentration or absorbance curves obtained during different dialysis therapies; and to offer objective parameters evaluating the differences between the ongoing and previous therapies. This problem is solved by a device with the features described in claim 1. Preferred embodiments of the invention are described in the claims 2 to 12.

Further goals, advantages, features and possibilities of use of this invention arise out of the subsequent description of the embodiments of the invention. Therefore every described or depict feature of its own or in arbitrary meaningful combination forms the subject matter of the invention even independent of its summary in the claims or its reference to other claims.

It shows:

FIG 1    Depicts a portion of a conventional dialysis machine plus a slight modification to host a sensor coupled with the dialysate circuit,

FIG 2    Shows an ongoing therapy concentration curve (solid line) together with a recorded concentration curve (dashed line).

FIG 3    Shows an ongoing therapy Kt/V curve (solid line) together with a recorded concentration curve (dashed line).

FIG 4    Shows a dialysis machine screen with a menu, which allows the user to select a recorded Kt/V curve.

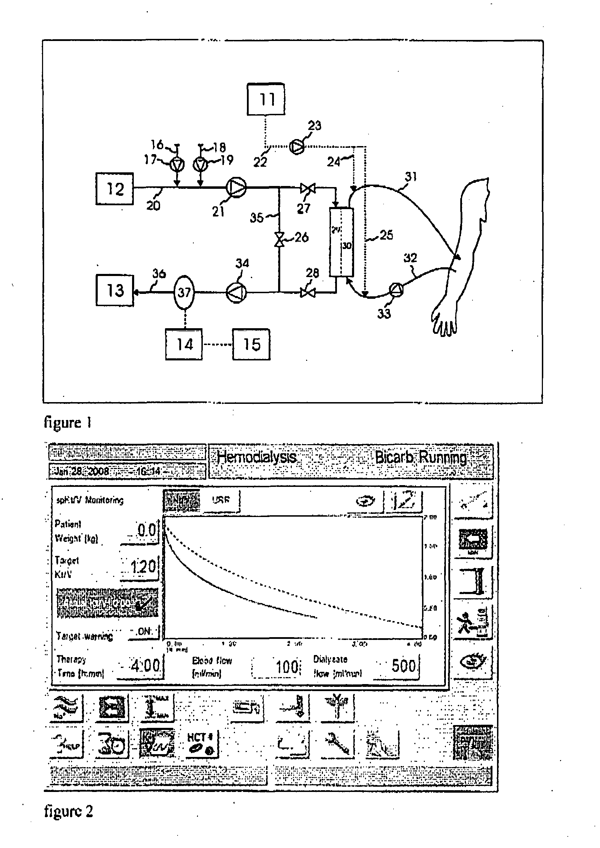

FIG. 1 shows a draw of the dialysate circuit of a conventional dialysis machine plus a slight modification to host a sensor coupled with the dialysate circuit. The blood from a patient is taken out into an extracorporcal circuit, it flows through the tube 32 into the blood chamber 30 of a dialyzer and returns to the patient through the tube 31. The flow rate of the blood circuit is controlled by the blood pump 33. The dialysis fluid is made of several concentrates and water, therefore the machine disclosed in figure 1 comprises a water inlet 12, two concentrates inlets 16 and 18 and two concentrate pumps 17 and 19.

The water flow together with the concentrates flow defines the final properties of the dialysis fluid. The conduit 20 takes the dialysis fluid to the dialysate chamber 29 of the dialyzer, which is separated from the blood chamber 30 by a semi permeable membrane.

The dialysis fluid it is pumped into the dialyzer by the pump 21. A second pump 34 sucks the dialysis fluid and any ultrafiltrate removed from the blood. A bypass line 35 is arranged between the pumps 21 and 34. Several valves 26, 27 and 28 are arranged to control the dialysate flow. The conduit 36 leads the spent dialysate to a UV-sensor 37 measuring its light absorbance, the UV-sensor 37 is connected by an interface with the computer 14 which processes the measured data, the result of the data processing is displayed and/or printed by the device 15, which is connected with the computer 14 by an interface. The conduit 36 leads the spent dialysate after its measurement by the U V-sensor 37 to the drain system 13. The dotted lines 22, 24 and 25 represent an adaptation of the disclosed apparatus for hemodiafiltration treatments. The substitution fluid comes from a substitution fluid source 11, flows through the line 22 and is pumped in the blood lines of the patient by the pump 23. In case of post dilution hemodiafiltration the conduit 24 leads the substitution fluid to the venous line of the extracorporeal blood system; in case of pre dilution hemodiafiltration the conduit 25 leads the substitution fluid to the arterial line of the extracorporeal blood system; and in case of pre-post dilution hemodiafiltration both conduits 24 and 25 are used. The computer 14 controls all the elements shown on the figure by means of proper interfaces, said interfaces arc not drawn for the sake of simplicity. The computer 14 gathers information about other parameters of the dialysis machine, like for example blood flow, dialysate flow and/or therapy time, these parameters together with the measured data are processed, the result tunes the Kt/V measuring functionality to assess deviations.

The UV-sensor 37 can be substituted by an Urea-sensor, in this case will the urea concentration in spent dialysate measured instead of the light absorbance. The disclosed dialysis machine is provided with several other means as is conventional. These other means arc not disclosed, since they are not relevant for the operation of the present invention.

The obtained absorbance curves are stored in a patient card attached to the computer 14 or in a database hosted in the computer 14. The number of curves which may be saved is variable and depends on the capacity of the storage medium. In our preferred embodiment the last 15 treatments are stored in a suitable patient card. The stored treatments are overwritten following a FIFO (first in first out) procedure. Additionally, a given therapy can be set as not over-writable, in such a case the therapy will be kept until it is set back to over-writable. It is also possible to store Kt/V or URR curves.

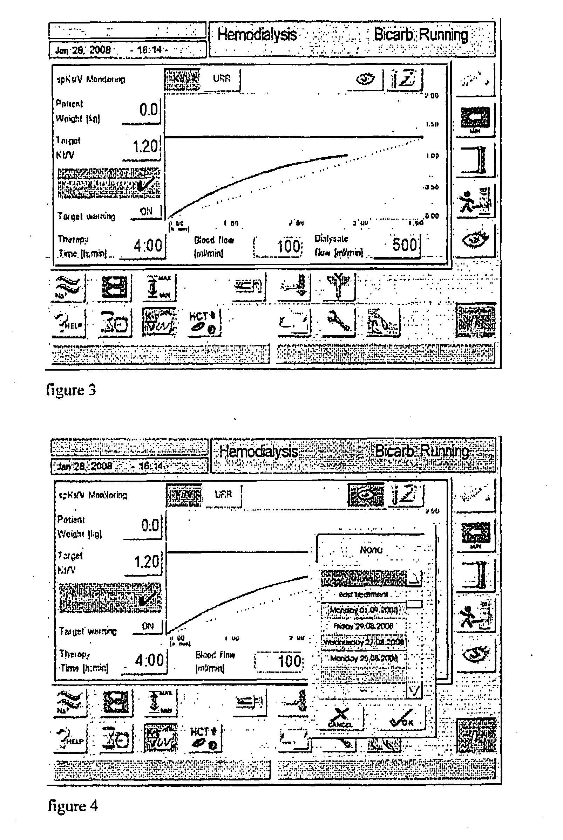

The different stored curves can be displayed by selecting them on a menu in the display of the dialysis machine (Fig. 4). In the preferred embodiment each curve is identified by the weekday and date when the therapy took place. The recorded curve is depicted below the currently ongoing curve with a lighter colour. The trend of the ongoing curve can be then visually compared with the previously recorded curve. One or more recorded curves can be displayed simultancously.

Specific curves can be identified by special names. In our preferred embodiment the treatment curve which delivered the best kt/V is saved as "Best treatment"; and the treatment which delivered the worst Kt/V is saved as "Worst treatment". These specific treatments can be kept out of the FIFO overwriting functionality, being only respectively overwritten if a better or worse treatment is recorded. The tracking of specific treatments may be reset by user action or after a predefined amount of time.

In our preferred embodiment an average curve out of all the curves stored in the patient card is calculate and identified as "average". This average curve is more valuable when comparing it against the ongoing curve because is less affected by extreme values and therefore more representative of the patient status.

The main factors affecting the shape of the absorbance curves are the blood flow and the dialysate flow. The stronger the counter current effect is, the more efficient the dialysis treatment is, and by extension the more steeper the UV-absorbance curve is. Ideally the recorded curves should be normalized to the ongoing treatment parameters to make them comparable.

Kety (Kety SS: Physiological and physical factors goveming the initial states of drug distribution, in Teorell T, Dedrick RL, Condliffe PG (eds): Pharmacology and Pharmacokinetics. New York, NY, Plenum Press, 1974, pp 233-240) developed a mathematical model describing the contribution of both blood flow and solute diffusion to solute transport across capillary membranes. This model relates dialyzer clearance with extracorporeal blood flow when applied to a dialyzer:

$$K = Q_b\left(1 - e^{-PS/Q_b}\right) \quad (1)$$

where $Q_b$ is the extracorporeal blood flow and PS is the product between dialyzer membrane permeability and membrane surface.

The mass balance of any compound during a dialysis therapy can be described as follows:

$$K \cdot C_b = Q_d \cdot C_d \quad (2)$$

where K is the dialyzer clearance; $C_b$ is The blood concentration; $Q_d$ is the dialysate flow, and $C_d$ is the dialysate concentration.

Because our goal is to normalize recorded treatments to the current treatment parameters regardless of the blood concentration, we can consider $C_b$, as a constant. Thus, it is possible to normalize each point of a recorded concentration curve to the ongoing curve as follows:

$$C_b = \frac{Q_{d_{recorded}} \cdot C_{d_{recorded}}}{K_{recorded}} ; \quad C_b = \frac{Q_{d_{current}} \cdot C_{d_{current}}}{K_{current}} \quad (3)$$

$$C_{d_{current}} = \frac{K_{current} \cdot Q_{d_{recorded}} \cdot C_{d_{recorded}}}{K_{recorded} \cdot Q_{d_{current}}} \quad (4)$$

Where $C_{d\text{-current}}$ is the normalized dialysate concentration; or in other words, the concentration that we would get in the recorded treatment if we had use the blood flow and dialysate flow of the current treatment.

By knowing the PS of the dialyzer it is possible to use equation I to calculate the K's, which are required by equation 4. It is also possible to use UV-absorbance values instead of concentrations in all the equations disclosed above.

In the preferred embodiment the PS factors four each dialyzer, type are available in the dialysis machine, new PS factors for other dialyzer types can be added at any time either by the technician servicing the machine or by the medical staff. The recorded therapy data includes the dialysate absorbance, blood flow and dialysate flow at each time point, and the PS factor. Before the start of the treatment the user selects the dialyzer. With the dialyzer selection and the current $Q_d$, the recorded curve is normalized to the current curve on real-time. If for example during the first half of a four hours therapy, the patient is treated with a K of 240 ml/min (300 ml/min blood flow) and during the second half with a K of 180 ml/min (200 ml/min blood flow), the recorded curve will be normalize to 240 ml/min for 2 hours and to 180 ml/min for 2 hours.

The normalization of the recorded curves to the current one is not mandatory. The dialysis machine offers the user the possibility to switch between two modes: normalized and not normalized.

During a dialysis procedure the dialysate flow prescription may change, such a change has important influence on the plotted concentration due to dilution effeets. An increase of dialysate flow from 400 ml/min to 800 ml/min will cause a 50% drop of the measured concentration, which can be misleading. It is important to account for dilution effects during a treatment. In our preferred embodiment the plotted concentration is on real time normalized to a standard dialysate flow of 500 ml/min. The normalization is achieved with the following equation:

$$C_n = C \cdot \left( \frac{Q_{d\text{-current}}}{Q_{d\text{-std}}} \right) = C \cdot \left( \frac{Q_{d\text{-current}}}{500} \right) \quad (5)$$

Where $C_n$ is normalized concentration; C is concentration; $Q_{d\text{-std}}$ is the standard dialysate flow to which the concentration is normalized; and $Q_{d\text{-current}}$ is the current dialysate flow. The concentrations may be substituted by UV-absorbance values.

The dilution effects can be illustrated by the following example. Let's suppose that the treatment is started with a dialysate flow of 500 ml/min and the first absorbance value is 3; after one hour the flow is set to 400 and the obtained absorbance is 3.1; furthermore, after two hours the flow is set to 800 ml/min and the obtained absorbance is 1.3. The normalized absorbance at each of the time points will be:

$$A_{n,t=0} = 3 \cdot \left( \frac{500}{500} \right) = 3$$

$$A_{n,t=1h} = 3.1 \cdot \left( \frac{400}{500} \right) = 2.5$$

$$A_{n,t=2h} = 1.3 \cdot \left( \frac{800}{500} \right) = 2.09$$

This approach avoids misleading the medical staff.

A visual comparison between recorded and ongoing treatments supposes a valuable help for the experience physician, however an objective parameter that evaluates the difference between the compared therapies would help on taking objective decisions regarding the current treatment or future prescriptions. In the preferred embodiment the difference between the area under the curve between the recorded and the current therapy is calculated. The integration of the concentration curve may be or not multiplied by the dialysate flow, in any of the cases it gives an idea of the amount of substances that were cleared from the patient. The difference between the two therapies quantifies how much more (or less) substances were removed.

**Claims**

1. A kidney substitution treatment machine for normalizing, displaying and storing curves describing the efficiency of a kidney substitution treatment or concentrations during said kidney substitution treatment,

   with an extracorporeal blood system (31, 32),

   with a dialyzer, which is divided by a semi-permeable membrane into a blood chamber (30) and a dialyzing fluid chamber (29) with means (33) to adjust a flow rate of the blood at a preset blood flow rate to pump the patient blood through a blood chamber (30) of the dialyzer,

   with means (21, 34) to adjust a flow rate of a dialyzing fluid at a preset flow rate through the dialyzing fluid system of the machine wherein the dialyzing fluid collects the waste products from the patient after flowing through the dialyzing fluid chamber (29) of the dialyzer and

   with one means (37) to measure continuously any kidney substitution treatment related waste product concentration to deliver together with the data provided by the kidney substitution treatment machine an adequacy parameter wherein the means (37) is coupled with the outlet conduit (36) of the dialyzing fluid system of the kidney substitution treatment machine and

   with means (14) to generate curves or functions of the adequacy parameter, with means to store curves or functions of the adequacy parameter, with means (15) to display curves or functions of the adequacy parameter,

   **characterised in that** the means (14) to generate curves or functions of the adequacy parameter is configured to normalize the curves of the adequacy parameter of the kidney substitution treatment to make them comparable, and the means to store curves or functions of the adequacy parameter is configured to store them in adequate media (14) and/or the means (15) to display curves or functions of the adequacy parameter is configured to display them on a user interface (15) of the machine delivering the kidney substitution treatment.

2. The kidney substitution treatment machine according to claim 1, with means (14) to calculate parameters quantify the adequacy differences between treatments.

3. The kidney substitution treatment machine according to claim 1 or 2, wherein the kidney substitution treatment is double needle hemodialysis, single needle hemodialysis, single needle cross over hemodialysis, post-dilution hemodiafiltration, pre-dilution hemodiafiltration, pre-post-dilution hemodiafiltration, post-dilution hemofiltration, predilution hemofiltration, pre-post-dilution hemofiltration or sequential hemodialysis.

4. The kidney substitution treatment machine according to one of the claims 1 to 3, wherein the stored curves are concentration, absorbance or UV-absorbance of any waste product present on the dialyzing fluid of any kidney substitution treatment.

5. The kidney substitution treatment machine according to one of the claims 1 to 4, wherein the stored curves are KUV, single pool Kt/V or equilibrated Kt/V of any waste product present on the dialyzing fluid of any kidney substitution treatment.

6. The kidney substitution treatment machine according to one of the claims 1 to 5, wherein the stored curves are the reduction ratio of any waste product present on the dialyzing fluid of any kidney substitution treatment, the single pool reduction ratio of any waste product present on the dialyzing fluid of any kidney substitution treatment, or the equilibrated reduction ratio of any taste - product present on the dialyzing fluid of any kidney substitution treatment.

7. The kidney substitution treatment machine according to one of the previous claims, wherein the means (37) to measure continuously any kidney substitution treatment related waste product is a UV-sensor.

8. The kidney substitution treatment machine according to one of the previous claims, wherein the storage media (14) is a hard disk hosted in the kidney substitution treatment machine, a memory card hosted in the kidney substitution treatment machine or a patient card attached to the kidney substitution treatment machine.

9. The kidney substitution treatment machine according to one of the previous claims according to one of the previous claims, wherein the stored curves are normalized to a given blood flow and/or a given clearance and/or a given dialysate flow.

10. The kidney substitution treatment machine according to one of the previous claims, wherein the difference between therapies is quantified by an algorithm comparing the area under the curve of the considered curves.

11. The kidney substitution treatment machine according to one of the previous claims, wherein one or more stored curves are shown simultaneously.

12. The kidney substitution treatment machine according to claim 11, wherein one best curve, one worst curve and one average curve are stored and/or displayed.

**Patentansprüche**

1. Ein Gerät zur Nierenersatzbehandlung zur Normierung, Darstellung und Speicherung von Kurven, die die Effizienz einer Nierenersatzbehandlung oder Konzentrationen während besagter Nierenersatzbehandlung beschreiben,
   mit einem extrakorporalen Blutsystem (31, 32),
   mit einem Dialysator, welcher durch eine semi-permeable Membran in eine Blutkammer (30) und eine Dialyseflüssigkeitskammer (29) geteilt ist mit Mitteln (33) zur Einstellung einer Flussrate des Blutes auf eine voreingestellte Blutflussrate um das Patientenblut durch eine Blutkammer (30) des Dialysators zu pumpen,
   mit Mitteln (21, 34) zur Einstellung einer Flussrate einer Dialyseflüssigkeit auf eine voreingestellte Flussrate durch das Dialyseflüssigkeitssystem des Gerätes, wobei die Dialyseflüssigkeit die Abfallprodukte aus dem Patienten nach dem Fließen durch die Dialyseflüssigkeitskammer (29) des Dialysators aufnimmt und
   mit einem Mittel (37) zur kontinuierlichen Messung der Konzentration eines jedweden Nierenersatzbehandlungsbezogenen Abfallsproduktes um zusammen mit den von dem Gerät zur Nierenersatzbehandlung gelieferten Daten einen Adäquanz-Parameter bereitzustellen, wobei das Mittel (37) an die Ausflussleitung (36) des Dialyseflüssigkeitssystems des Gerätes zur Nierenersatzbehandlung gekoppelt ist und
   mit einem Mittel (14) um Kurven oder Funktionen des Adäquanz-Parameters zu generieren, mit einem Mittel zur Speicherung von Kurven oder Funktionen des Adäquanz-Parameters, mit einem Mittel (15) zur Darstellung von Kurven oder Funktionen des Adäquanz-Parameters,
   **dadurch gekennzeichnet, dass** das Mittel (14) um Kurven oder Funktionen des Adäquanz-Parameters zu generieren dazu konfiguriert ist die Kurven des Adäquanz-Parameters der Nierenersatzbehandlung zu normieren um sie vergleichbar zu machen, und das Mittel zur Speicherung der Kurven oder Funktionen des Adäquanz-Parameters dazu konfiguriert ist sie in einem geeigneten Medium (14) zu speichern und/oder das Mittel (15) zur Darstellung von Kurven oder Funktionen des Adäquanz-Parameters dazu konfiguriert ist sie auf einer Benutzeroberfläche (15) des die Nierenersatzbehandlung durchführenden Gerätes darzustellen.

2. Das Gerät zur Nierenersatzbehandlung gemäß Anspruch 1, mit einem Mittel (14) zur Berechnung von Parametern zur Quantifizierung der Adäquanz-Unterschiede zwischen Behandlungen.

3. Das Gerät zur Nierenersatzbehandlung gemäß Anspruch 1 oder 2, wobei es sich bei der Nierenersatzbehandlung um Zwei-Nadel-Hämodialyse, Einzelnadel-Hämodialyse, Einzelnadel-Cross-Over-Hämodialyse, Hämodiafiltration mit Postdilution, Hämodiafiltration mit Prädilution, Hämodiafiltration mit Prä-Post-Dilution, Hämofiltration mit Postdilution, Hämofiltration mit Prädilution, Hämofiltration mit Prä-Post-Dilution oder sequentielle Hämodialyse handelt.

4. Das Gerät zur Nierenersatzbehandlung gemäß einem der Ansprüche 1 bis 3, wobei es sich bei den gespeicherten Kurven um Konzentration, Absorption oder UV-Absorption eines jedweden Abfallproduktes in der Dialyseflüssigkeit einer jedweden Nierenersatzbehandlung handelt.

**5.** Das Gerät zur Nierenersatzbehandlung gemäß einem der Ansprüche 1 bis 4, wobei es sich bei den gespeicherten Kurven um KUV, single-pool Kt/V oder äquilibrierter Kt/V eines jedweden Abfallproduktes in der Dialyseflüssigkeit einer jedweden Nierenersatzbehandlung handelt.

**6.** Das Gerät zur Nierenersatzbehandlung gemäß einem der Ansprüche 1 bis 5, wobei es sich bei den gespeicherten Kurven um die Reduktionsrate eines jedweden Abfallproduktes in der Dialyseflüssigkeit einer jedweden Nierenersatzbehandlung, die Single-Pool-Reduktionsrate eines jedweden Abfallproduktes in der Dialyseflüssigkeit einer jedweden Nierenersatzbehandlung, oder die äquilibrierte Reduktionsrate eines jedweden Abfallproduktes in der Dialyseflüssigkeit einer jedweden Nierenersatzbehandlung handelt.

**7.** Das Gerät zur Nierenersatzbehandlung gemäß einem der vorherigen Ansprüche, wobei es sich bei dem Mittel (37) zur kontinuierlichen Messung eines jedweden Nierenersatzbehandlungs-bezogenen Abfallproduktes um einen UV-Sensor handelt.

**8.** Das Gerät zur Nierenersatzbehandlung gemäß einem der vorherigen Ansprüche, wobei es bei dem Speichermedium (14) um eine in dem Gerät zur Nierenersatzbehandlung untergebrachte Festplatte, eine in dem Gerät zur Nierenersatzbehandlung untergebrachte Speicherkarte oder eine an dem Gerät zur Nierenersatzbehandlung befestigte Patientenkarte handelt.

**9.** Das Gerät zur Nierenersatzbehandlung gemäß einem der vorherigen Ansprüche, wobei die gespeicherten Kurven auf einen vorgegebenen Blutfluss und/oder eine vorgegebene Clearance und/oder einen vorgegebenen Dialysatfluss normiert werden.

**10.** Das Gerät zur Nierenersatzbehandlung gemäß einem der vorherigen Ansprüche, wobei der Unterschied zwischen Therapien durch einen Algorithmus quantifiziert wird, der die Fläche unter der Kurve der betrachteten Kurven vergleicht.

**11.** Das Gerät zur Nierenersatzbehandlung gemäß einem der vorherigen Ansprüche, wobei eine oder mehrere gespeicherte Kurven gleichzeitig angezeigt werden.

**12.** Das Gerät zur Nierenersatzbehandlung gemäß Anspruch 11, wobei eine beste Kurve, eine schlechteste Kurve und eine gemittelte Kurve gespeichert und/oder dargestellt werden.

## Revendications

**1.** Un appareil de traitement de substitution rénale pour normaliser, afficher et stocker des courbes décrivant l'efficacité d'un traitement de substitution rénale ou les concentrations pendant ledit traitement de substitution rénale,
avec un système du sang extracorporel (31, 32),
avec un dialyseur, qui est séparé par une membrane hémiperméable dans une chambre sanguine (30) et une chambre de liquide de dialyse (29), avec un moyen (33) pour ajuster le débit du sang à un débit du sang préréglé pour pomper le sang du patient à travers la chambre sanguine (30) du dialyseur,
avec des moyens (21, 34) d'ajustement du débit du liquide de dialyse à un débit préréglé à travers le système de liquide de dialyse de l'appareil, dans lequel le liquide de dialyse collecte les produits de déchet provenant du patient après circulation à travers la chambre du liquide de dialyse (29) du dialyseur et avec un moyen (37) de mesure continue de la concentration de n'importe quel produit de déchet associé au traitement de substitution rénale pour fournir un paramètre d'adéquation en même temps que les données fournies par l'appareil de traitement de substitution rénale, dans lequel le moyen (37) est couplé avec le conduit de sortie (36) du système de liquide de dialyse de l'appareil de traitement de substitution rénale et
avec un moyen (14) de génération des courbes ou des fonctions du paramètre d'adéquation,
avec un moyen de stockage des courbes ou des fonctions du paramètre d'adéquation,
avec un moyen (15) d'affichage des courbes ou des fonctions du paramètre d'adéquation,
**caractérisé en ce que** le moyen (14) de génération des courbes ou des fonctions du paramètre d'adéquation est configuré pour normaliser les courbes du paramètre d'adéquation du traitement de substitution rénale afin de les rendre comparables, et
le moyen de stockage des courbes ou des fonctions du paramètre d'adéquation est configuré pour les stocker sur un milieu adéquat (14) et/ou le moyen (15) d'affichage des courbes ou des fonctions du paramètre d'adéquation est configuré pour les afficher sur une interface utilisateur (15) de l'appareil fournissant le traitement de substitution rénale.

2. L'appareil de traitement de substitution rénale selon la revendication 1, avec un moyen (14) pour calculer les paramètres quantifiant les différences d'adéquation entre les traitements.

3. L'appareil de traitement de substitution rénale selon la revendication 1 ou 2, dans lequel le traitement de substitution rénale est l'hémodialyse double-aiguille, l'hémodialyse simple-aiguille, l'hémodialyse «cross-over» simple-aiguille, l'hémodiafiltration post-dilution, l'hémodiafiltration pré-dilution, l'hémodiafiltration pré-post-dilution, l'hémofiltration post-dilution, l'hémofiltration pré-dilution, l'hémofiltration pré-post-dilution ou l'hémodialyse séquentielle.

4. L'appareil de traitement de substitution rénale selon une des revendications 1 à 3, dans lequel les courbes stockées sont la concentration, l'absorbance ou l'absorbance UV de n'importe quel produit de déchet présent dans le liquide de dialyse de n'importe quel traitement de substitution rénale.

5. L'appareil de traitement de substitution rénale selon une des revendications 1 à 4, dans lequel les courbes stockées sont les KUV, les Kt/V single pool ou les Kt/V équilibrés de n'importe quel produit de déchet présent dans le liquide de dialyse de n'importe quel traitement de substitution rénale.

6. L'appareil de traitement de substitution rénale selon une des revendications 1 à 5, dans lequel les courbes stockées sont le taux de réduction de n'importe quel produit de déchet présent dans le liquide de dialyse de n'importe quel traitement de substitution rénale, le taux de réduction single pool de n'importe quel produit de déchet présent dans le liquide de dialyse de n'importe quel traitement de substitution rénale, ou le taux de réduction équilibré de n'importe quel produit de déchet présent dans le liquide de dialyse de n'importe quel traitement de substitution rénale.

7. L'appareil de traitement de substitution rénale selon une des revendications précédentes, dans lequel le moyen (37) de mesure continue de n'importe quel produit de déchet associé à n'importe quel traitement de substitution rénale est un senseur UV.

8. L'appareil de traitement de substitution rénale selon une des revendications précédentes, dans lequel le milieu de stockage (14) est un disque dur hébergé par l'appareil de traitement de substitution rénale, une carte mémoire hébergée par l'appareil de traitement de substitution rénale ou une carte de patient attachée à l'appareil de traitement de substitution rénale.

9. L'appareil de traitement de substitution rénale selon une des revendications précédentes, dans lequel les courbes stockées sont normalisées à un débit donné du sang et/ou à une clairance donnée et/ou à un débit du dyalisat donné.

10. L'appareil de traitement de substitution rénale selon une des revendications précédentes, dans lequel la différence entre les thérapies est quantifiée par un algorithme qui compare l'aire au-dessous de la courbe des courbes considérées.

11. L'appareil de traitement de substitution rénale selon une des revendications précédentes, dans lequel une ou plusieures courbes stockées sont montrées simultanément.

12. L'appareil de traitement de substitution rénale selon la revendication 11, dans lequel une meilleure courbe, une pire courbe et une courbe moyenne sont stockées et/ou montrées.

figure 1

figure 2

figure 3

figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1083948 B1

### Non-patent literature cited in the description

- Solute transport mechanisms in dialysis. **DEPNER T ; GARRED I..** Replacement of renal function by dialysis. Kluwer academic publishers, 2004, 73-91
- Hemodialysis machines and monitoris. **POLASCHE-GG HD ; LEVIN NW.** Replacement of renal function by dialysis. Kluwer academic publishers, 2004, 414-418
- **I. FRIDOLIN ; M. MAGNUSSON ; L-G. LINDBERG.** On-line monitoring of solutes in dialysate using absorption of ultraviolet radiation: Technique description. *The International Journal of Artificial Organs,* 2002, vol. 25 (8), 748-761

- **UHLIN F.** Haemodialysis treatment monitored online by ultra violet absorbance. Linköping University Medical Dissertations n° 962. *Department of Medicine and Care Division of Nursing Science & Department of Biomedical Engineering,* 2006
- Physiological and physical factors goveming the initial states of drug distribution. **KETY SS.** Pharmacology and Pharmacokinetics. Plenum Press, 1974, 233-240